(19) Europäisches Patentamt
European Patent Office
Office européen des brevets

(11) **EP 4 400 592 A2**

(12) **EUROPEAN PATENT APPLICATION**

(43) Date of publication:
**17.07.2024 Bulletin 2024/29**

(21) Application number: **24178175.6**

(22) Date of filing: **08.05.2023**

(51) International Patent Classification (IPC):
**C12N 15/65** (2006.01)

(52) Cooperative Patent Classification (CPC):
**C12N 15/65; C12N 15/85;** C12N 2830/002

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.05.2022 US 202217747589**

(62) Document number(s) of the earlier application(s) in accordance with Art. 76 EPC:
**23171982.4 / 4 279 592**

(71) Applicant: **Sartorius Stedim Biotech GmbH 37079 Göttingen (DE)**

(72) Inventors:
• **Akbari, Samin**
  **Winchester, MA (US)**
• **Leroux, Ann-Cathrin**
  **Neu-Ulm (DE)**
• **Kwon, Taehong**
  **Cambridge, MA (US)**
• **Zehe, Christoph**
  **Ehingen (DE)**
• **Pollard, David**
  **Stow, MA (US)**

(74) Representative: **Müller-Boré & Partner Patentanwälte PartG mbB Friedenheimer Brücke 21 80639 München (DE)**

Remarks:
This application was filed on 27.05.2024 as a divisional application to the application mentioned under INID code 62.

(54) **CELL-BASED SHEAR STRESS SENSOR**

(57) The present invention relates to a method of evaluating fluid shear stress acting on cells in real-time in a bioprocess and/or during the operation of a biotechnological device or system, comprising the steps of subjecting a cell to said bioprocess and/or to operation of said biotechnological device or system, wherein said cell comprises an isolated nucleic acid molecule or a nucleic acid vector comprising said isolated nucleic acid molecule, wherein said isolated nucleic acid molecule comprises a functional mammalian EGR-1 (early growth response protein 1) promoter region that is operatively linked to a gene encoding a fluorescent protein, or a gene encoding an siRNA directed against a constitutively expressed fluorescent protein, and controls expression of said gene; determining a level of fluid shear stress acting on cells during said bioprocess and/or during operation of said biotechnological device or system using said cell in real-time at any given time, and determining said level of fluid shear stress as the level of fluid shear stress that is acting on any cells subjected to said bioprocess and/or to operation of said biotechnological device or system at said given time.

EP 4 400 592 A2

## Description

The present invention relates to isolated nucleic acid molecules, comprising a functional mammalian EGR-1 (early growth response protein 1) promoter region that is operatively linked to a gene encoding either a fluorescent protein or a gene encoding an siRNA directed against a constitutively expressed fluorescent protein and controls expression of said gene. The present invention further relates to nucleic acid vectors, comprising said nucleic, cells comprising said nucleic acids or vectors, being capable of sensing and indicating fluid shear stress acting on themselves, and methods of generating the same. Furthermore, the present invention relates to methods of evaluating fluid shear stress acting on cells in real-time during the operation of a biotechnological device or system, methods of evaluating a biotechnological device or system with respect to fluid shear stress acting on cells caused by operation of said device or system, and methods of designing a biotechnological device or system, said methods using said cells.

Biological and biotechnological application using live cells in one way or the other inherently require fluid handling and, thus, are prone to less-than-optimal performance due to fluid shear stress acting on said cells. Fluid shear stress can be caused by fluid shear, as well as bursting bubbles in a fluid, and can be responsible for all kinds of detrimental effects such as impaired cell growth and expansion, impaired cell health, increased cell damage and/or cell death, impaired cell productivity, and the like.

In particular, shear stress during bioreactor cultivation has significant impact on cell health, growth, and fate. T cells and stem cells in next-generation cell therapies are especially more sensitive to shear stress present in their culture environment. Therefore, a base knowledge about the shear stress imposed by the bioprocesses is needed to optimize the process parameters and enhance cell growth and yield.

Understanding and measuring fluid shear stress in stirred tank bioreactors is crucial in bioreactor development and process scale-up. Fluid shear stress results from mechanical agitation through impellers and gas bubbles arising from aeration. Its magnitude depends on many factors such as rotational speed of the impeller, geometry of the bioreactor vessel and impeller, and viscosity of cell culture. Since excessive shear stress could reduce the growth and viability of various cell lines used in bioreactor cultivation, it is important to predict and measure shear stress accurately to operate bioreactors within the stress tolerance level of cells.

However, fluid shear stress applied to cells in respective bioprocesses, such as in bioreactors, is so far only poorly understood. The common practice to evaluate fluid shear stress is to perform computational flow dynamics to model the flow, which is challenging for a turbulent three-phase (air, media and cells) fluidic system. In addition, modeling often requires many simplifications in terms of cell size, shape, concentration, culture viscosity, *etc.*, which cannot be representative of real bioreactor cultivation. Furthermore, interpretation of the results is often complicated (*e.g.*, which part of the bioreactor is representative of shear stress, whether to minimize average or maximum shear stress). Another approach for shear stress characterization is to use PCR methods to measure levels of gene expression of cells under shear stress. Despite its robust and precise measurement, PCR-based assay has some limitations such as cumbersome sample preparation (*e.g.*, nucleic acid extraction), time delay, and high cost (equipment, skills, reagents, *etc.*). Further, assays assessing cell viability, cell morphology and/or cell growth can be employed to detect more severe physiological disturbances caused by fluid shear stress. However, these assays are too general and do not specifically target the pathways induced by shear stress. There are various causes for changes in viability and morphology besides shear stress (*e.g.*, lack of nutrients, accumulation of toxic byproducts or lack in oxygen).

Thus, so far, no assay exists that could evaluate the effects of fluid shear stress acting on cells within the setting of a given biological or biotechnological application.

Therefore, a strong need exists to provide means for evaluating fluid shear stress acting on cells within a given biological or biotechnological application quantitively and in real-time.

This need is satisfied by providing the embodiments characterized in the claims.

The present invention relates to the following items:

1. An isolated nucleic acid molecule, comprising a functional mammalian EGR-1 (early growth response protein 1) promoter region that is operatively linked to

    (i) a gene encoding a fluorescent protein, or
    (ii) a gene encoding an siRNA directed against a constitutively expressed fluorescent protein,

and controls expression of said gene.

2. The isolated nucleic acid molecule according to item 1, wherein the EGR-1 promoter region is a human EGR-1 promoter region.

3. The isolated nucleic acid molecule according to item 1, wherein the EGR-1 promoter region is a murine EGR-1 promoter region.

4. The isolated nucleic acid molecule according to item 3, wherein the EGR-1 promoter region comprises the 527 base pairs of the native murine EGR-1 promoter upstream to its transcription start site.

5. The isolated nucleic acid molecule according to item 3, wherein the EGR-1 promoter region comprises the 425 base pairs of the native murine EGR-1 promoter upstream to its transcription start site.

6. The isolated nucleic acid molecule according to any one of items 1 to 5, wherein the fluorescent protein is selected from the group consisting of green fluorescent protein (GFP), derivatives of GFP, red fluorescent protein (RFP), and derivatives of RFP.

7. The isolated nucleic acid molecule according to item 6, wherein derivatives of GFP are selected from the group consisting of GFPmut2, and EGFP.

8. The isolated nucleic acid molecule according to item 6, wherein a derivative of RFP is TurboRFP.

9. A nucleic acid vector, comprising the nucleic acid according to any one of items 1 to 8.

10. A cell, comprising the nucleic acid according to any one of item 1 to 8.

11. The cell according to item 10, which is a CHO cell, a HEK cell, a T cell, a stem cell, or an endothelial cell.

12. The cell according to item 10, which is a CHO DG44 cell.

13. The cell according to item 10, which is a HEK293 cell.

14. A cell, comprising the vector according to item 9.

15. The cell according to item 14, which is a CHO cell, a HEK cell, a T cell, a stem cell, or an endothelial cell.

16. The cell according to item 14, which is a CHO DG44 cell.

17. The cell according to item 14, which is a HEK293 cell.

18. The cell according to item 16, comprising the vector according to item 9, said vector comprising the nucleic acid according to item 6, wherein the fluorescent protein is GFP, and wherein said cell further expresses an IgG antibody.

19. The cell according to item 18, wherein the antibody is an $IgG_1$ antibody.

20. A method of generating a cell that is capable of indicating fluid shear stress acting on said cell, comprising the step of transfecting a cell with the nucleic acid according to any one of items 1 to 8.

21. The method according to item 20, wherein the cell is a CHO cell, a HEK cell, a T cell, a stem cell, or an endothelial cell.

22. The method according to item 20, wherein the cell is a CHO DG44 cell.

23. The method according to item 20, wherein the cell is a HEK293 cell.

24. A method of generating a cell that is capable of indicating fluid shear stress acting on said cell, comprising the step of transfecting a cell with the vector according to item 9.

25. The method according to item 24, wherein the cell is a CHO cell, a HEK cell, a T cell, a stem cell, or an endothelial cell.

26. The method according to item 24, wherein the cell is a CHO DG44 cell.

27. The method according to item 24, wherein the cell is a HEK293 cell.

28. A method of evaluating fluid shear stress acting on cells in real-time during the operation of a biotechnological device or system, comprising the steps of:

    (a) subjecting a cell according to any one of items 10 to 19 to operation of said biotechnological device or system,
    (b) determining a level of fluid shear stress acting on cells during operation of said biotechnological device or system using said cell in real-time at any given time, and
    (c) determining said level of fluid shear stress as the level of fluid shear stress that is acting on any cells subjected to operation of said biotechnological device or system at said given time.

29. A method of evaluating a biotechnological device or system with respect to fluid shear stress acting on cells caused by operation of said biotechnological device or system, comprising the steps of:

    (a) providing a biotechnological device or system,
    (b) subjecting a cell according to any one of items 10 to 19 to operation of said biotechnological device or system,
    (c) determining a level of fluid shear stress acting on cells during operation of said biotechnological device or system using said cell, and
    (d) in case the level of fluid shear stress determined in step (c) is higher than a predetermined acceptable level of fluid shear stress, evaluating said biotechnological device or system as unfavorable with respect to fluid shear stress acting on cells, and in case the level of fluid shear stress determined in step (c) is lower than said predetermined acceptable level of fluid shear stress, evaluating said biotechnological device or system as favorable with respect to fluid shear stress acting on cells.

30. A method of designing a biotechnological device or system, comprising the steps of:

(a) providing a candidate biotechnological device or system,
(b) subjecting a cell according to any one of items 10 to 19 to operation of said candidate biotechnological device or system,
(c) determining a level of fluid shear stress acting on cells during operation of said candidate biotechnological device or system using said cell, and
(d) in case the level of fluid shear stress is higher than a predetermined acceptable level of fluid shear stress, modifying the design of the candidate biotechnological device or system and repeating steps (a) to (d) until the level of fluid shear stress determined in step (c) is lower than said predetermined acceptable level of fluid shear stress, wherein upon each repetition of steps (a) to (d), the new candidate biotechnological device or system used in step (a) uses the previously modified biotechnological device or system design.

The figures show:

Figure 1 :
GFP fluorescence of the cells, measured by Incucyte (Live cell imaging and analysis instrument, Sartorius). Phorbol 12-myristate 13-acetate (PMA) at different concentrations was used to induce cellular stress. PMA was added at 0 hour. (Upper panel) Fluorescent signal over monitoring period. The error bars represent the standard deviation of eight technical replicates. (Lower panel) Normalized GFP fluorescence at different PMA concentrations at 24 h (No PMA = 1).

Figure 2:
Shear stress response at different magnitudes and durations. Fluorescence intensity 24 hours after shear stress application was used. The response was normalized with respect to no shear stress. Error bars represent the standard deviation of the data within the independent three experiments. (Upper panel) GFP fluorescence response of the cells at different magnitudes of shear stress. (Lower panel) The response at different durations of shear stress.

Figure 3:
Shear response of cells in a commercial small-scale bioreactor at different power densities and culture durations. Stirring speed was varied to change power density. Fluorescence was normalized by control culture flasks ($n = 2$) on a shaker. The values in this figure were obtained from five technical replicates, and the coefficient of variance (CV) for these replicates was 4.5% $\pm$ 3.9%. (Upper panel) The plot of power density (x axis) vs. shear stress response (y axis). (Lower panel) The plot of cultivation time (x axis) vs. shear stress response (y axis).

Figure 4:
Cellular shear stress response in two different bioreactors (vessel A and B) at different power densities and culture durations. Fluorescence was normalized by control culture flasks ($n = 2$) on a shaker. The average of 5 technical replicates was used for each condition. The values in this figure were obtained from five technical replicates, and the coefficient of variance (CV) for these replicates was 6.0% $\pm$ 4.2%.

Figure 5:
Inducibility of cell pools stably overexpressing GFP under control of EGR-1 promoter (inducible, LP3-6) and SF40 promoter (constitutive, LP1-2). Pools were induced with 100 nM PMA. GFP fluorescence was measured at 18 h post transfection. Data was normalized to GFP fluorescence of uninduced pools.

Figure 6:
Plasmid used for the generation of stress inducible stable CHO cell lines. The EGR1 promoter mediates stress inducibility.

Figure 7:
Inducibility of generated stress inducible clones over a cultivation period of 35 days. Cells were treated after two days of cultivation with 100 nM PMA. GFP fluorescence intensity was measured after an incubation period of 17-18 hours by flow cytometry (iQue® 3, Sartorius).

Figure 8:
GFP fluorescence of the cells under chemical (PMA) induction. The fluorescence was measured by flow cytometer.

Figure 9:
Application of fluid shear stress to the CHO cell-based sensor using a microfluidic chip and monitoring of shear response of the sensor by IncuCyte® (Sartorius). (A) Example of shear stress response from the sensor. Two different shear stress conditions were applied to the sensor and its fluorescence signal was monitored by Incucyte over 48 hours. "No flow" is data from the sensor that experienced no shear condition. Error bars in the control are standard deviation ($n$ = 3; three independent control channels). (B) The cells exhibiting fluorescence after application of fluid shear stress (60 min at 21.2 dyn/cm$^2$) in the microfluidic chip. The cell image was acquired by IncuCyte® at 36 hours.

In a first aspect, the present invention relates to an isolated nucleic acid molecule, preferably an isolated DNA molecule, comprising a functional mammalian EGR-1 (early growth response protein 1) promoter region, preferably a functional human or murine EGR-1 promoter region, that is operatively linked to (i) a gene encoding a fluorescent protein, or (ii) a gene encoding an siRNA directed against a constitutively expressed fluorescent protein, and controls expression of said gene.

EGR-1 is a mammalian transcription factor that functions as a transcriptional regulator. The expression of EGR-1 is controlled by the EGR-1 promoter which is responsive to various stimuli, including various forms of cell stress. As used herein, the term "functional EGR-1 promoter region" refers to any EGR-1 promoter regions that are capable of (i) being activated by such stimuli and (ii) initiating transcription of the gene downstream of the promoter upon such activation. Likewise, the term "operatively linked to a gene" refers to the same capability of initiating transcription of said gene upon activation of the promoter region.

In specific embodiments, the EGR-1 promoter region comprises the 527 base pairs of the native murine EGR-1 promoter upstream to its transcription start site. In another specific embodiment, the EGR-1 promoter region comprises the 425 base pairs of the native murine EGR-1 promoter upstream to its transcription start site.

Fluorescent proteins that can be used in the context of the present invention are not particularly limited and are known in the art. In specific embodiments, the fluorescent protein is selected from the group consisting of green fluorescent protein (GFP), derivatives of GFP (such as GFPmut2, EGFP (Enhanced GFP)), red fluorescent protein (RFP), and derivatives of RFP (such as TurboRFP).

In the case of an isolated nucleic acid molecule of the present invention in which the EGR-1 promoter region is operatively linked to a gene encoding a fluorescent protein, shear stress acting on cells comprising said nucleic acid molecule results in an increase of expression of the fluorescent protein, i.e., an increase in the respective fluorescence that can be measured for said cells. In contrast, in the case of an isolated nucleic acid molecule of the present invention in which the EGR-1 promoter region is operatively linked to a gene encoding an siRNA directed against a constitutively expressed fluorescent protein, shear stress acting on cells comprising said nucleic acid molecule results in a decrease of expression of the fluorescent protein, i.e., a decrease in the respective fluorescence that can be measured for said cells.

In this context, use of an siRNA directed against a constitutively expressed fluorescent protein can improve response dynamics by way of (i) decreasing time from stress signal to response signal, and (ii) speeding up reporter degradation. In particular, a respective siRNA can bind to a constitutively expressed fluorescent protein with short turnover times. In this case, decreasing fluorescence intensity implies an increase in shear stress. This siRNA approach can reduce response time, as protein expression is bypassed.

In a second aspect, the present invention relates to a nucleic acid vector, comprising the nucleic acid according to the present invention. Respective vectors are not particularly limited and are known in the art. They include for example suitable expression vectors known in the art. Nucleic acid vectors according to the present invention can comprise additional genetic elements such as genes encoding one or more selection markers, polyadenylation signal sequences, and enhancers, as known in the art.

In a third aspect, the present invention relates to a cell, comprising the nucleic acid according to the present invention, or the nucleic acid vector according to the present invention. Respective cells function as a sensor of fluid shear stress, i.e., they are capable of indicating fluid shear stress acting upon them. Specifically, upon fluid shear stress acting on said cells, the EGR-1 promoter region is activated and initiates transcription of (i) the gene encoding the fluorescent protein, resulting in the expression of said fluorescent protein which can be detected by suitable means for the detection of fluorescence known in the art, detecting an increase in fluorescence, or (ii) the gene encoding an siRNA directed against a constitutively expressed fluorescent protein, resulting in the expression of said siRNA which down-regulates expression of a constitutively expressed fluorescent protein which can be detected by suitable means for the detection of fluorescence known in the art, detecting a decrease in fluorescence.

Suitable cell types that can be used according to the present invention are not particularly limited and include any cells that might be of interest in this respect. In specific embodiments, the cell is a CHO (Chinese hamster ovary) cell (e.g. a CHO DG44 cell), a HEK (human embryo kidney) cell (e.g. a HEK293 cell), a T cell, a stem cell, or an endothelial cell.

Means of transfecting a given cell with the nucleic acid or nucleic acid vector of the present invention are not particularly limited and are known in the art.

In a fourth aspect, the present invention relates to a method of generating a cell that is capable of indicating fluid shear stress acting on said cell, comprising the step of transfecting a cell with the nucleic acid according to the present invention, or with the nucleic acid vector according to the present invention.

In this aspect, the cell is as defined for the third aspect of the present invention. Further, as indicated above, means of transfecting a given cell with the nucleic acid or nucleic acid vector of the present invention are not particularly limited and are known in the art.

In a fifth aspect, the present invention relates to a method of evaluating fluid shear stress acting on cells in real-time during the operation of a biotechnological device or system, comprising the steps of:

> (a) subjecting a cell according to the present invention to operation of said biotechnological device or system,
> (b) determining a level of fluid shear stress acting on cells during operation of said biotechnological device or system using said cell in real-time at any given time, and
> (c) determining said level of fluid shear stress as the level of fluid shear stress that is acting on any cells subjected to operation of said biotechnological device or system at said given time.

As used herein the term "in real-time" refers to the fact that according to the above method, fluid shear stress acting on cells during operation of the biotechnological device or system can be detected and evaluated while operation of the biotechnological device or system is ongoing. Further, as used herein, the term "biotechnological device or system" relates to any devices or systems that employ or act on living cells in any way or form at least at some time during their intended operation. By way of example, respective biotechnological devices include bioreactors, microfluidic devices and systems, flow cytometry devices and systems, cell culture devices, cell culture devices and systems, cell manipulation devices and systems, cell analysis devices and systems and the like, as known in the art.

The term "subjecting a cell to operation of said biotechnological device or system" refers to any use of the cells of the present invention in the context of the intended operation of said device or system. This can be an exclusive use of the cells of the present invention, or a combined use together with cells of interest in the context of the intended operation of the device or system. In case of the latter, the combined use can be in the form of mixing the cells of the present invention with the cells of interest, or in the form of a separate use of the cells of the present invention during the intended operation of the device or system, e.g. in a suitable culture vessel or live cell imaging device, provided that the cells of the present invention are subjected to the same potential fluid shear stress as the cells of interest.

As used herein, the term "determining a level of fluid shear stress acting on cells during operation of said biotechnological device or system using said cell" refers to the measurement of fluorescence signal emitted by the fluorescent protein (i) potentially expressed by the cells of the present invention, or (ii) potentially downregulated by the cells of the present invention, and correlating the same to an amount of fluid shear stress. As indicated above, respective means of detecting/measuring fluorescence are not particularly limited and are known in the art.

In a related sixth aspect, the present invention relates to a method of evaluating a biotechnological device or system with respect to fluid shear stress acting on cells caused by operation of said biotechnological device or system, comprising the steps of:

> (a) providing a biotechnological device or system,
> (b) subjecting a cell according to the present invention to operation of said biotechnological device or system,
> (c) determining a level of fluid shear stress acting on cells during operation of said biotechnological device or system using said cell, and
> (d) in case the level of fluid shear stress determined in step (c) is higher than a predetermined acceptable level of fluid shear stress, evaluating said biotechnological device or system as unfavorable with respect to fluid shear stress acting on cells, and in case the level of fluid shear stress determined in step (c) is lower than said predetermined acceptable level of fluid shear stress, evaluating said biotechnological device or system as favorable with respect to fluid shear stress acting on cells.

In this aspect, all definitions for the method according to the fifth aspect of the present invention equally apply.

In a related seventh aspect, the present invention relates to a method of designing a biotechnological device or system, comprising the steps of:

> (a) providing a candidate biotechnological device or system,
> (b) subjecting a cell according to any one of items 10 to 17 to operation of said candidate biotechnological device or system,
> (c) determining a level of fluid shear stress acting on cells during operation of said candidate biotechnological device or system using said cell, and
> (d) in case the level of fluid shear stress is higher than a predetermined acceptable level of fluid shear stress, modifying the design of the candidate biotechnological device or system and repeating steps (a) to (d) until the level of fluid shear stress determined in step (c) is lower than said predetermined acceptable level of fluid shear stress, wherein upon each repetition of steps (a) to (d), the new candidate biotechnological device or system used in step (a) uses the previously modified biotechnological device or system design.

In this aspect, all definitions for the method according to the fifth aspect of the present invention equally apply.

As used herein, the term "candidate biotechnological device or system" refers to a biotechnological device or system according to a proposed design. In step (d) of the above method, this design is either accepted as the final design of the biotechnological device or system, in case the level of fluid shear stress determined in step (c) is below an acceptable level, or the design is modified in a way that is potentially alleviating fluid shear stress during operation of the device or system, in case the level of fluid shear stress determined in step (c) is above an acceptable level. This modified biotechnological device or system is then subjected to a new round of analysis according to the above method.

The present invention uses genetically modified cells that respond to shear stress in the form of expressing or downregulating fluorescent proteins. Cells that experience higher magnitude or duration of shear stress will generate either a higher or lower signal that can be quantified. Thus, the present invention provides a useful tool to characterize the true shear stress applied to cells in bioprocesses including bioreactors, tubing, pumps, etc., as well as in the evaluation, characterization and design of new biotechnological devices or systems and bioprocessing equipment.

The cell-based sensors of the present invention advantageously report the true impact by the whole shear stress acting on said cells, thus indicating what cells feel like and go through due to the combination of shear stresses.

The present invention will be further illustrated in the following examples without any limitation thereto.

**Examples**

Material and methods:

*Sensor fabrication*

A CHO-DG44 clone expressing $IgG_1$ antibody was transfected with a plasmid carrying the stress inducible EGR-1 promoter with GFP as a reporter molecule. EGR-1 promoter has been previously described to be sheer stress sensitive. Stable cell pools were generated by neomycin selection. PMA (PMA, Sigma, P1585-1MG) at a concentration of 100 nM was added to the cells and GFP fluorescence was measured 18 h post induction using flow cytometry (IqueScreener iQue® 3, Sartorius). The pool with the largest shift in GFP fluorescence was used for single cell cloning via FACS (BD FACS Aria Fusion, BD biosciences). 120 clones were screened for PMA stress responsiveness and the top 4 clones were selected. The top 4 clones were passaged every 3 days for 35 days in 125 mL shake flasks (36.8°C, 7.5% $CO_2$).

*Cell culture in shake flasks*

CHO DG44 cells expressing GFP under control of stress-inducible EGR-1 were cultured in media composed of 4Cell® XtraCHO Stock & Adaptation Medium (SAM) (Sartorius, Germany), 6 mM L-Glutamine (Corning, NY, USA), 15 nM MTX (Pfizer Pharma, PZN-3362608) and 250 µg/mL G418 (Gibco, 10131027). Cells were cultivated by standard method, briefly, $1 \times 10^7$ cells were dispersed in 50 mL media to achieve inoculation concentration of $2 \times 10^5$ cells/mL in a 250 mL shake flask (4115-0250, Erlenmeyer flask, Thermo Scientific). Cells were incubated at 36.8 °C with 7.5 % $CO_2$ supplement and an orbital shaking at 103 RPM and passaged every 3 days. For the stability study, the cells were cultivated on a linear shaker at 110 rpm.

*Sensor characterization - chemical (PMA) induced stress*

Chemical-induced stress was studied using Phorbol 12-myristate 13-acetate (PMA, Sigma, P1585-1MG) and measured by Incucyte (Sartorius) and flow cytometer (CytoFLEX, Beckman Coulter; iQue 3, Sartorius). PMA was used to mimic short-term fluid shear stress because of its upregulation of EGR-1. PMA was dissolved in DMSO to make a stock solution of 0.5 mg/mL. For Incucyte measurements, cells ($1 \times 10^4$ cells/well) were seeded in a 96 well plate. PMA was added after 3 h of seeding to achieve final concentration of 0, 10, 100, 250, and 500 ng/mL in the wells. Upon addition of PMA, the plate was scanned (4 images per well) under GFP mode with $10\times$ objective every 1 h for 3 days. Exposure time was set to be 100 ms. Mean GFP fluorescent intensity of each cell were obtained by integrated GFP intensity normalization by cell area per image. For flow cytometer measurements, cells ($2 \times 10^5$ cells/well) were seeded in 24 well plate. Cell concentration reached $5 \times 10^5$ cells/mL after 3 days of cultivation. PMA were added subsequently to achieve final concentration of 0, 10, 100, 250, and 500 ng/mL in the wells. After 24 h, cells were collected by centrifugation (190 g, 3 min), washed 3 times with PBS and dispersed in PBS to measure GFP intensity by flow cytometry. For sensor characterization during stability assessment, 200 µL of a 2-day old culture was transferred to a 96-well plate and incubated with 100 nM PMA at 36.8°C and 7.5% $CO_2$. After 17-18 h, GFP intensity was measured by flow cytometry (iQue 3, Sartorius) directly on the incubated 96-well plate.

*Sensor characterization - fluid shear stress*

Commercial microfluidic chips (µ-Slide VI 0.4, 80604, ibidi; height 400 um, width 3.8 mm, length 17 mm) were used to apply fluidic shear stress to the CHO cells. The channel surface had poly-L-Lysine for cell attachment. 30 µL of CHO cells at $0.75 \times 10^6$ cells/mL were seeded to the microfluidic channel and incubated in the 7.5% $CO_2$ incubator for 60 min for cell attachment. Subsequently, the tubes from the medium reservoirs were connected to the microfluidic channel through Luer-lock connection in a sterile manner. Then, the pressure pump (PG-MFC-8CH, PreciGenome) gave an oscillatory flow to the microfluidic channel at a target flow rate; the flow rate was controlled by varying pressures. The fluidic shear stress on the cells in the channel was calculated according to the following equation:

$$\tau = \eta \cdot 176.1 \cdot \Phi$$

with $\tau$ shear stress (dyn/cm$^2$), $\eta$ dynamic viscosity (dyn·s/cm$^2$), and $\Phi$ flow rate (mL/min). The dynamic viscosity of culture medium was assumed to have 0.0068 dyn·s/cm$^2$ (water at 37°C). Following shear stress application at different magnitudes (0, 8.0, 12.9, and 21.9 dyn/cm$^2$) and duration (10, 30, and 60 min), the culture medium reservoirs were disconnected from the microfluidic chip. The microfluidic chips were then placed on a tray in the live cell imaging and analysis instrument (Incucyte®, Sartorius) in the 7.5% CO$_2$ incubator. The instrument captured phase contrast and fluorescent images at a regular time interval (60 min) and analyzed them automatically (cell identification and signal intensity quantification). For quantification of cellular shear-stress response, the green fluorescence of the cells at 24 hours after shear stress application was averaged and normalized by the fluorescence of the cells in the no-flow (i.e., no shear stress) channels.

*Sensor test in Ambr bioreactors*

Two different types of commercial bioreactors (Ambr® 250, Sartorius) were tested: vessel A (001-2A23, baffled vessel, two impellers, 26 mm impeller diameter, Sartorius) and vessel B (001-2A33, unbaffled vessel, one impeller, 30 mm impeller size, Sartorius). The power number of the impeller for two vessels was 1.34 and 2.07, respectively. Other culture flasks (4115-0250, Erlenmeyer flask, Thermo Scientific; 658195, Suspension culture flask, Greiner) were used as controls. The shear-responsive cells were cultured in shake flasks in a 7.5% CO$_2$ incubator on a batch mode (see Cell culture section above). After 3-day cultivation, the cells were inoculated into Ambr bioreactors (vessel A and B) at $0.3 \times 10^6$ cells/mL (viability: >98%). The working volume of the bioreactor was 120 to 165 mL. The bioreactor medium consisted of SMD, L-Glutamine (6 mM), MTX (15 nM), and G418 (250 μg/mL). Feed A, Feed B, and Anti-Foam C (2 v/v%), concentrated glucose (400 g/L; 97062-946, VWR) were prepared in separate reservoirs, which were connected to the bioreactor. The pH and DO were set to 7.1 and 60%, respectively. They were automatically controlled by an Ambr station (Ambr 250 modular, Sartorius) with gases (O$_2$, N$_2$, and CO$_2$). Temperature was set to 36.8 °C. Different agitation RPMs (1000, 1500, 2000, and 2500) were applied to the bioreactors. The agitation power per unit volume (P/V, W/m$^3$; power density) was calculated as follows:

$$\frac{P}{V} = \frac{\left( N_p \times \rho \times N^3 \times D^5 \right)}{V}$$

with $N_p$ impeller power number, $\rho$ fluid density (kg/m$^3$; 1000 for water), $N$ agitator speed (rps), D impeller outer diameter (m), and V the bioreactor's working volume (m$^3$). The other control flasks were placed on a shaker (Dura-Shaker, VWR) and agitated at 108 rpm speed. The culture samples from Ambr 250 bioreactors and control flasks were taken at different time points (0 hr, 4 hr, 1 day, 3 days, and 5 days). After cell concentration was counted by the automated cell culture analyzer (Cedex HiRes Analyzer, Roche), the sampled cells were seeded into the 96 well plate at $0.04 \times 10^6$ cells/mL concentration (volume: 150 μL). Subsequently, the plate was placed in Incucyte in the incubator, and fluorescent shear response of the cells was automatically monitored at a regular time interval (60 min). For quantification of cellular shear response, the fluorescence signal at 24 hours after seeding was used. The mean fluorescence intensity of the cells sampled from the bioreactors was normalized by the intensity of the cells from the control flasks.

Example 1:

Sensor characterization - PMA induced stress

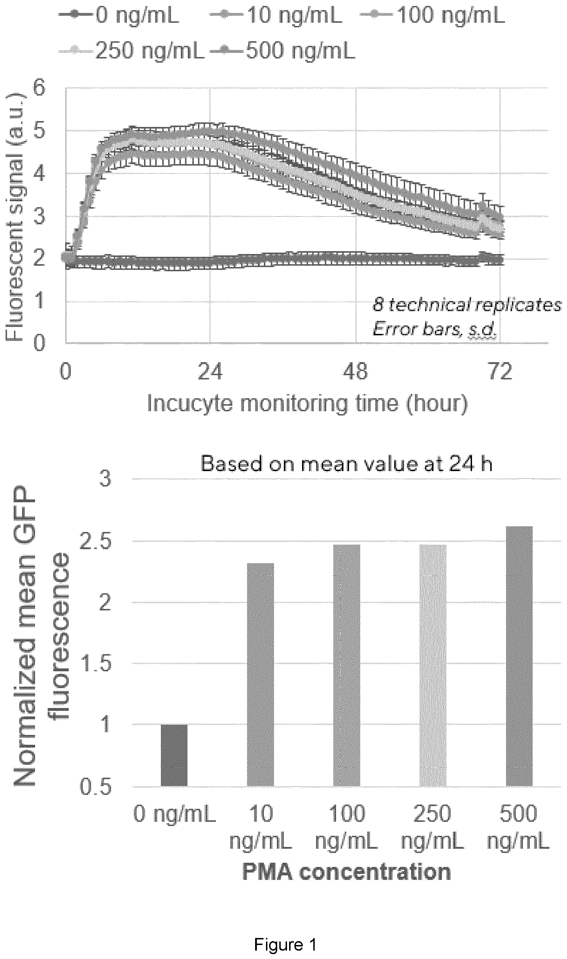

PMA is known for upregulating EGR-1 and used to mimic short-term fluid shear stress. GFP fluorescence measured by Incucyte is shown in Figure 1. 0 ng/mL PMA only shows basal GFP fluorescence intensity in the full recording course (72 h). 10 ng/mL PMA induces lower level of increased GFP fluorescence intensity compared with 100 ng/mL, 250 ng/mL and 500 ng/mL PMA. GFP fluorescence intensity increases and plateaus in the first 10 h and decreases from 24 h after PMA addition. Fold GFP fluorescence intensity change normalized with 0 ng/mL PMA at 24 h was 2.32, 2.46, 2.47, 2.61 for 10, 100, 250, 500 ng/mL PMA, respectively.

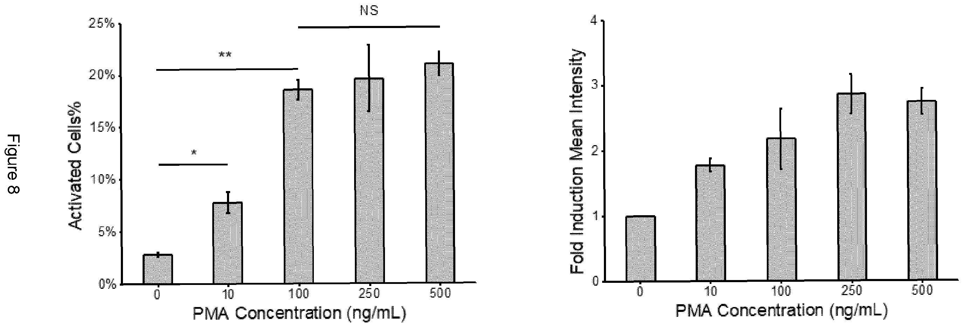

GFP fluorescence measured by flow cytometer showed that percent of activated cells were 2.79%, 7.83%, 18.55%, 19.65% and 21% for 0, 10, 100, 250, 500 ng/mL PMA, respectively (Fig. 8). Fold GFP fluorescence intensity change normalized with 0 ng/mL PMA was 1.78, 2.17, 2.86, 2.74 for 10, 100, 250, 500 ng/mL PMA, respectively.

Example 2:

Sensor characterization - Fluid shear stress (microfluidic chip + Incucyte)

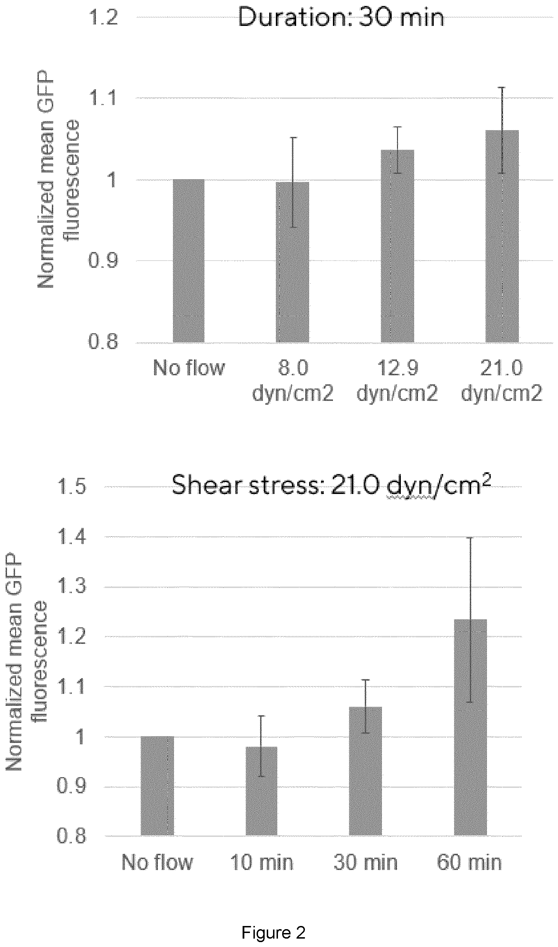

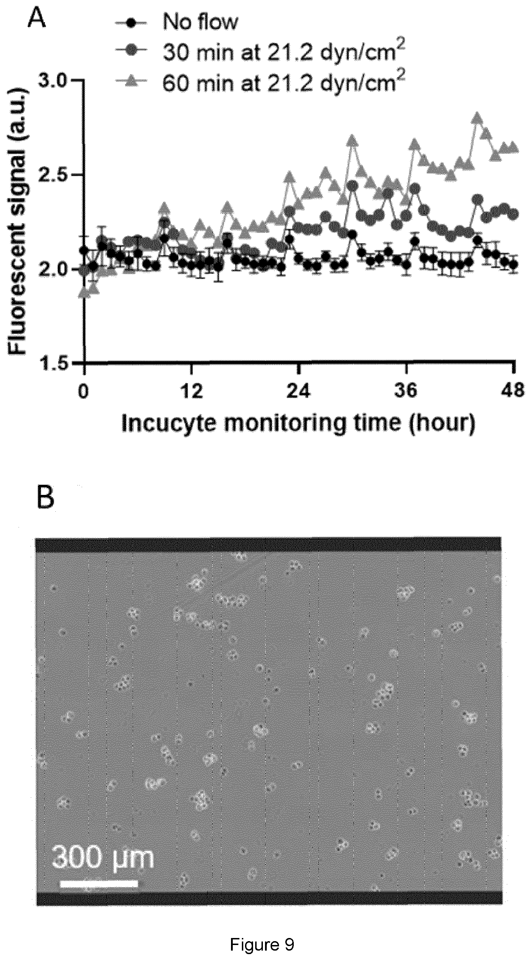

As shown in Figure 9, microfluidic channels and Incucyte® were used to characterize the fluidic shear response of the sensor CHO cells. Fluidic shear stress was applied to the cells in the microfluidic channels. The cells were under oscillatory flow at different magnitudes and durations. Figure 2 shows the shear stress response at different magnitudes (8.0, 12.9, and 21.0 dyn/cm$^2$) at a fixed duration (30 min). The fluorescence level of the cells at the lowest shear stress 8.0 dyn/cm$^2$ was similar to the no-flow (*i.e.*, no shear stress) control. However, further increase in stress magnitude to 12.9 and 21.0 dyn/cm$^2$ had higher fluorescence. The shear stress level 21.0 dyn/cm$^2$ produced the highest fluorescence. Increase in mean fluorescence was 6%.

In addition to stress magnitude, different durations of shear stress were tested. The shear stress level was fixed to 21.0 dyn/cm$^2$. Compared to the cells in no-shear-stress channels, the normalized shear stress levels of three different durations were 0.98, 1.06, and 1.23, respectively. The longer duration of shear stress application caused more fluorescent responses from the cells.

Example 3:

Sensor test in Ambr reactors

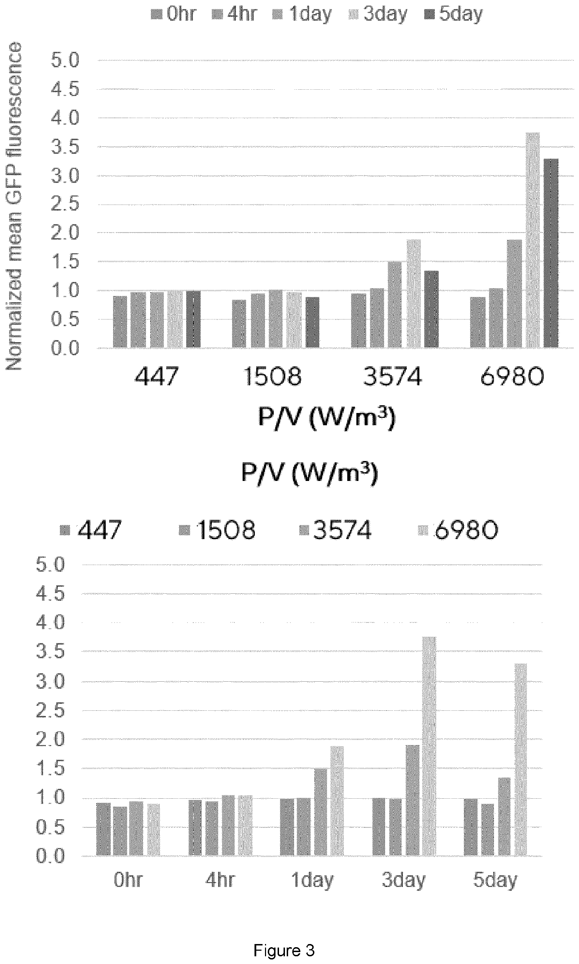

The shear response of the cells was tested in a commercial small-scale bioreactor ("vessel A", power number 1.34, baffled, two impellers, impeller outer diameter 26 mm) under different shear stress conditions. Although the shear stress is not homogenous in the bioreactor setting, the average fluorescence response of the sensor cells can be representative of the shear stress magnitude and time sensed by the cells. By varying stirring speeds, different power densities (power per unit volume; P/V in W/m$^3$) were obtained. Four power densities (447, 1508, 3574, and 6980 W/m$^3$) were tested at different culture durations (0 hr, 4 hr, 1 day, 3 days, and 5 days), as shown in Figure 3. The power densities of 447 and 1508 did not increase shear stress response regardless of culture duration. However, the increase in cellular response was observed starting 3574 W/m$^3$ and after 24-hour cultivation. The higher power density (6980 W/m$^3$) produced more response of the cells.

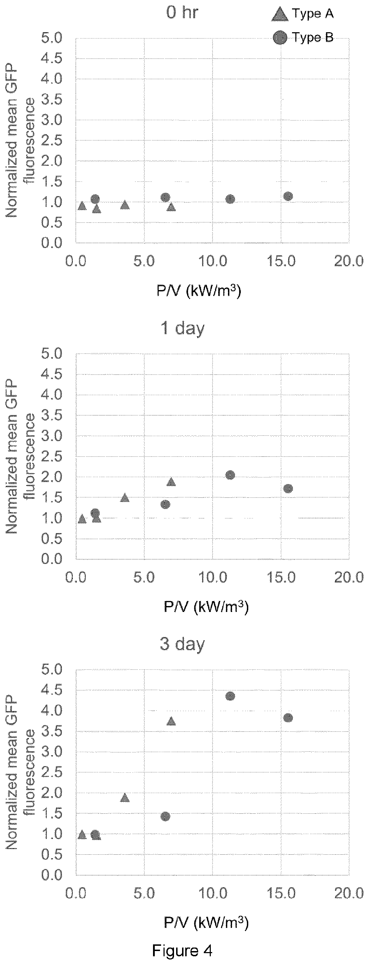

The cell-based shear stress sensor can be applied toother bioreactors for comparison of shear stress levels in different bioreactors. The cells were cultured in another bioreactor vessel with different impellers ("Vessel B", power number 2.07, unbaffled, one impeller, impeller outer diameter 30 mm). Figure 4 shows the cellular response in two different bioreactors (vessel A and B) at different power densities and culture durations. Different bioreactor conditions and impeller structure produced different power densities. Compared to 0 hr, both vessel A and B bioreactors during 1-day cultivation exhibited the increase in shear stress response at higher P/V. Vessel A bioreactor had 1.5 response at 3574 P/V; Vessel B bioreactor had 1.33 response at 6550 P/V. Higher signal intensity (1.89) of vessel A than vessel B (1.33) in a similar P/V range (6550 to 6980 W/m$^3$) indicates vessel A caused more shear stress on the cells than vessel B. 3-day cultivation further supports this. At a similar P/V (6550 to 6980 W/m$^3$), vessel B had smaller shear stress response than vessel A (2.62-fold difference). As P/V increased further above 10 kW/m$^3$, the vessel B bioreactor showed a similar high shear stress response to vessel A at 1-day and 3-day cultivation, indicating that cultivation in two bioreactors at this agitation condition is not suitable for cell cultivation.

Example 4:

Sensor fabrication

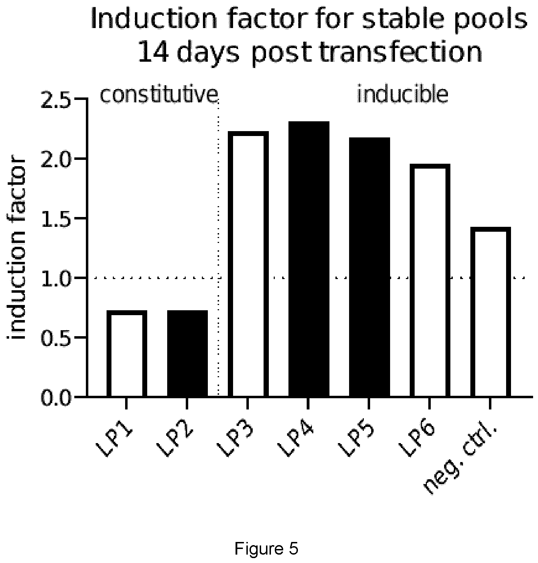

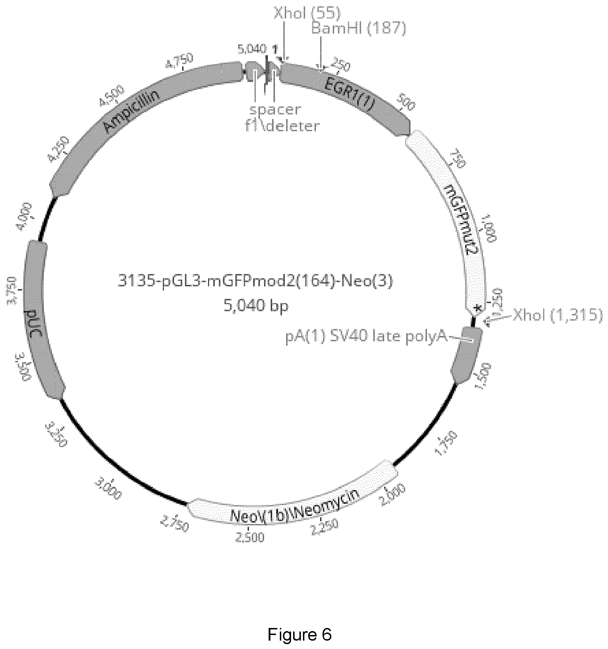

Stable CHO cell pools (LPs) that express GFP under the control of the EGR-1 promoter (stress inducible) and SV40 promoter (constitutive) were generated (Figs. 5 and 6). Upon PMA induction, fluorescence intensity increased about 2-fold. The pools with the highest dynamic range upon PMA stress induction (black bars in Fig. 5) were used for the generation of 120 single cell clones to identify clones with higher dynamic range in stress responsiveness compared to their host pools.

Example 5:

Sensor characterization - stability test

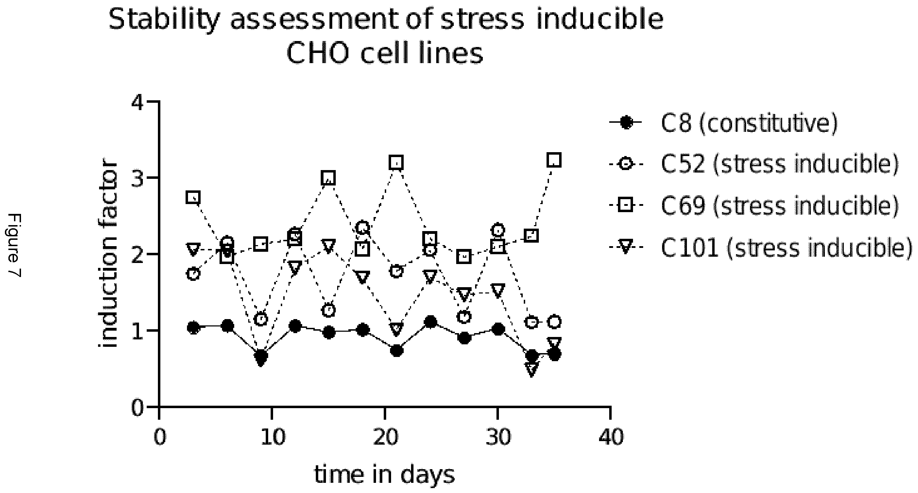

Stability of sheer stress inducibility of the cell line is critical for assessment of shear stress in a bioprocess environment. The top three shear-stress-inducible clones were tested for stability of stress inducibility. Three clonal cell lines stably expressing GFP under the EGR1-promoter (stress inducible) and one clonal cell line expressing GFP constitutively (non-inducible) were cultivated over 35 days in batch culture. GFP expression was induced with PMA every three days in a separate cultivation dish. The induction factor indicates the fold change increase of GFP fluorescence intensity with vs. without PMA induction (Fig. 7). Generally, there is a fluctuation of the induction factor visible over time. The clones and their host pools had the similar average stress inducibility. Since clone 69 showed the highest PMA inducibility over time, it was chosen for the sheer stress evaluation of different Ambr® 250 bioreactor vessels.

Discussion:

The present invention demonstrates a cell-based shear stress sensor for bioreactor cultivation. A CHO-DG44 clone containing shear stress-inducible EGR-1 promoter and GFP was developed. Its sensitivity and stability was studied using chemical induction and fluid shear stress. Finally, demonstration of this cell-based sensor in the actual Ambr® 250 bioreactor showed that it could assess sheer stress imposed by different bioreactors and operating conditions.

A stable CHO cell line with GFP expression under the control of stress inducible EGR-1 promoter was developed. The GFP reporter gene expression of PMA-induced cells exhibited about 2- to 2.5-fold increase, compared to uninduced cells. It was shown that the sensor response peaked at 17-24 hours post PMA induction followed by a 48-hour phase of GFP fluorescence signal reduction to levels comparable to baseline.

Flows in the microfluidic channels were used to characterize the sensitivity and operating range of the sensors before the test in bioreactors. The maximum shear stress and duration tested in the microfluidic experiments were 21.0 dyn/cm$^2$ and 60 minutes, respectively, (Fig. 2). Considering higher fluorescence intensity of the sensors from the bioreactor experiments at harsh agitation conditions (Fig. 3), the sensors could detect higher magnitude and longer duration of shear stress. Testing these conditions in the microfluidic channel was challenging in the current study because of cell detachment from the channel surface at these intense shear conditions. Further optimization of bonding conditions (e.g., different coating materials and conditions) could enable testing of intense fluid conditions in the microfluidic channel and make it a useful tool for shear stress calibration.

The shear stress levels of two different Ambr® 250 vessels (type A and B) were evaluated using the sensors. Both vessels exhibited higher shear stress levels at higher power density and longer duration (Fig. 4). However, type A had a steeper power density-sensor response curve than type B, indicating type A has a higher shear stress level given the similar power density. In fact, type B is a Sartorius' new Ambr® 250 bioreactor that is unbaffled and has a single elephant ear-shaped impeller. Its unique configuration provided more gentle mixing for T cells and human mesenchymal stem cells on beads and microcarriers than the baffled and double-impeller bioreactor (as in Type A), thus improving cell production. Although the sensor reported high shear stress level at intense power densities (tested up to 6,980 W/m$^3$ for Type A and 15,525 W/m$^3$ for Type B; corresponding to 2000 stirring rpm), it is noted that the normal operating power densities are significantly lower than these shear response-triggering power densities. For example, 855 rpm giving 279 W/m$^3$ is commonly used for regular CHO cell cultivation using Type A vessel without causing shear stress.

In the present invention, a CHO-DG44 cell line-based shear stress sensor was developed and tested in commercial small-scale bioreactors. The stress-sensitive-promoter EGR-1 in the engineered CHO cells expresses GFP upon shear stress. Using chemically induced and fluidic shear stress, stable expression of GFP by the sensor was confirmed for more than a month and its sensitivity and operating range examined. Following this characterization, the shear stress level of the commercial small-scale bioreactors was evaluated under different vessel designs and operating conditions (stirring speed and duration). The sensor expressed more GFP in response to higher stress magnitude and exposure time under harsh agitation conditions. No shear stress response was observed under typical bioprocess conditions at less than 1000 rpm agitation.

## Claims

1. A method of evaluating fluid shear stress acting on cells in real-time in a bioprocess and/or during the operation of a biotechnological device or system, comprising the steps of:

   (a) subjecting a cell to said bioprocess and/or to operation of said biotechnological device or system,

   wherein said cell comprises an isolated nucleic acid molecule or a nucleic acid vector comprising said isolated nucleic acid molecule,
   wherein said isolated nucleic acid molecule comprises a functional mammalian EGR-1 (early growth response protein 1) promoter region that is operatively linked to

   (i) a gene encoding a fluorescent protein, or
   (ii) a gene encoding an siRNA directed against a constitutively expressed fluorescent protein,

   and controls expression of said gene;

   (b) determining a level of fluid shear stress acting on cells during said bioprocess and/or during operation of said biotechnological device or system using said cell in real-time at any given time, and
   (c) determining said level of fluid shear stress as the level of fluid shear stress that is acting on any cells subjected to said bioprocess and/or to operation of said biotechnological device or system at said given time.

2. The method according to claim 1, wherein the EGR-1 promoter region is a human EGR-1 promoter region, or a murine EGR-1 promoter region.

3. The method according to claim 2, wherein the EGR-1 promoter region comprises the 527 base pairs of the native murine EGR-1 promoter upstream to its transcription start site, or the 425 base pairs of the native murine EGR-1 promoter upstream to its transcription start site.

4. The method according to any one of claims 1 to 3, wherein the fluorescent protein is selected from the group consisting of green fluorescent protein (GFP), derivatives of GFP, red fluorescent protein (RFP), and derivatives of RFP.

**5.** The method according to claim 4, wherein derivatives of GFP are selected from the group consisting of GFPmut2, and EGFP, and a derivative of RFP is TurboRFP.

**6.** The method according to any one of claims 1 to 5, wherein the cell is a CHO cell, a HEK cell, a T cell, or a stem cell.

**7.** The method according to claim 6, wherein the cell is a CHO DG44 cell, or a HEK293 cell.

**8.** The method according to any one of claims 1 to 7, wherein the cell is a CHO DG44 cell, comprising a nucleic acid vector comprising said nucleic acid molecule, wherein the fluorescent protein is GFP, and wherein said cell further expresses an IgG antibody.

**9.** The method according to claim 8, wherein the IgG antibody is an $IgG_1$ antibody.

**10.** The method according to any one of claims 1 to 9, wherein said cells are used in combination with cells of interest in the context of said bioprocess and/or the operation of said biotechnological device or system.

**11.** The method according to any one of claims 1 to 10, wherein said bioprocess involves the use of a bioreactor, tubing, and/or a pump.

Figure 1

Figure 2

Figure 3

Figure 4

Figure 5

Figure 6

Figure 7

Figure 8

Figure 9